# EUROPEAN PATENT APPLICATION

(11) **EP 3 087 937 A1**
(43) Date of publication of application: **02.11.2016**
(21) Application number: 16166951.0
(22) Date of filing: 25.04.2016
(51) Int. Cl.: A61B 17/74

(54) **SURGICAL SCREW WITH AN INVERTED THREAD FOR PERTROCHANTERIC FRACTURES ON THE LEFT SIDE**

(30) Priority: 28.04.2015 ES 201530477 U
(71) Applicant: Paya Zaforteza, Enrique Mateo, 46930 Valencia (ES)
(72) Inventor: PAYA ZAFORTEZA, ENRIQUE MATEO, 46930 VALENCIA (ES); PAYA ZAFORTEZA, ENRIQUE MATEO, 46930 VALENCIA (ES)
(74) Representative: Maldonado Jordan, Julia

(57) **Abstract**

The invention relates to a surgical screw with an inverted thread for pertrochanteric fractures on the left side comprising a body (1) having a series of notches made at one of its ends to allow performing a counterclockwise movement with an external tool for screwing it in, and having a thread (2) at the other end in the inverted direction, such that the body must be introduced counterclockwise.

## Description

### Object of the Invention

The object of the present utility model is a surgical screw with an inverted thread for pertrochanteric fractures on the left side, specifically as an insertion element in extramedullary osteosynthesis systems used for the osteosynthesis of stable pertrochanteric fractures of the proximal extremity of the femur on the left side.

### Background of the Invention

Fractures of the proximal extremity of the femur today have an annual incidence of between 200 and 400 cases out of every 1,000 people. About 50% of these are pertrochanteric fractures, and about half of these fractures are on the left side.

For decades now the most widely used system in pertrochanteric fractures of the proximal extremity of the femur has been based on a large screw inserted through a hole made in the outer cortex of the femur, reaching the femoral head through the femoral neck, said screw being referred to as a "lag screw." The stem of this screw is then inserted into a cylinder which is welded to a plate with holes for screws (said device commonly referred to as "cylinder plate." The plate is screwed in with several smaller screws (referred to as "cortical screws"), in direct contact with the outer face of the femoral diaphysis. The cylinder and the plate form an angle between 130º and 135º.

The stem of the lag screw can slide freely inside the plate cylinder, although the end of this stem is usually tightened with a small additional screw integrally joining both components.

The angle between the plate and the lag screw means that rotations of the fragments caused by the rotation of the lag screw occur on an axis between 130-135º with the vertical. In the case of pertrochanteric fractures, this axis is usually almost perpendicular to the plane of the fracture.

It must be taken into account that unlike in other types of surgical treatments, the focal point of the fracture is not approached directly in pertrochanteric fractures of the femur since the fragments are not directly handled with reduction forceps or other bone holding systems; it could therefore be considered a percutaneous technique. This procedure has the benefit of shortening the intervention, which is important in these patients who are elderly for the most part and have a high risk in relation to analgesia and surgery.

Therefore, one of the objectives in the treatment of patients of this type is early mobility to prevent the onset of complications due to being bedridden for a prolonged period, such as, for example, pressure ulcers and hypostatic pneumonia.

Another objective is the early rehabilitation and walking, which is achieved with aggressive physical therapy and allows the patients to rapidly return to the conditions prior to the accident.

During the surgical procedure one of the technical problems resulting from the techniques and elements used today is that the rotation of the screw makes the proximal fragment (femoral head and neck) rotate with it and this rotation in fractures on the right side causes the extension and internal rotation in the proximal fragment. This brings about better reduction of the fragments and bringing them closer to their reduced position, and therefore, improving the relative positions of the fragments.

In contrast, in fractures on the left side the situation is the opposite, and the rotation of the lag screw does not cause the extension and internal rotation of the proximal fragment, but rather flexion and external rotation. As a result, the proximal fragment becomes synthesized directly in the position of external rotation and flexion with respect to the distal fragment, and therefore when the patient starts to walk and support weight, the foot is turned inwardly. This causes a more unstable and uncomfortable gait for the patient and it can even cause the implant to fail or break.

### Description of the Invention

The technical problem to be solved by the present invention is to achieve a screw with an inverted thread that helps to produce extension and internal rotation of the proximal fragment. To that end, the surgical screw with an inverted thread for pertrochanteric fractures on the left side object of the present utility model is characterized by comprising a body characterized in that it has a thread in the inverted direction at one of its ends, such that the body must be introduced counterclockwise.

As a result of the inverted thread, in the screw herein disclosed the rotation of the lag screw will have the same beneficial effect both in fractures on the left side and in fractures on the right side. The effect on relative positions of the fragments is that it causes internal rotation and extension of the proximal fragment, counteracting the traction of the iliopsoas muscle and improving reduction of the fragments.

Accordingly, when patients start to walk again they will do so with their foot in the right position, will recover better and have greater functionality and independence, improving their quality of life and in turn reducing the risk of new falls.

The improvement in fractures on the left side that can be treated with this new screw will prevent fractures that may have been badly reduced and therefore reduce the associated economic cost that could result from poorly treated fractures which, in most cases, will lead to new interventions, with the problems this entails for patients.

Finally, the screw herein proposed, i.e., a screw with an inverted thread, will not involve an evident increase in the cost of manufacturing said screws and will improve the results obtained in the prognosis for survival and the quality of life of the patients treated with this new screw.

Throughout the description and claims the word "comprises" and variants thereof do not seek to exclude other technical features, additions, components or steps. For the persons skilled in the art, other objects, advantages and features of the invention can be inferred in part from the description and in part from practice of the invention. The following examples and drawings are provided by way of illustration and are not intended to limit the present invention. Furthermore, the present invention covers all the possible combinations of particular and preferred embodiments herein indicated.

### Brief Description of the Drawings

A series of drawings that help to better understand the invention and that are expressly related with an embodiment of said invention, presented as a non-limiting example thereof, will be very briefly described below.

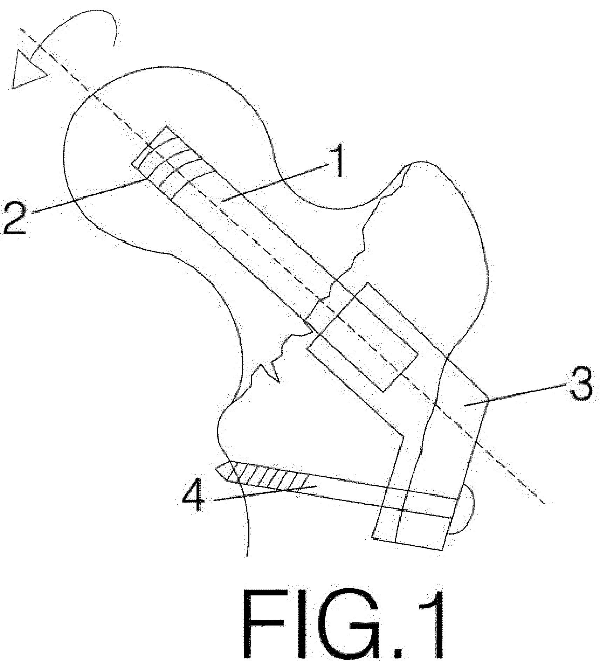

Figure 1 shows a view of the surgical screw with an inverted thread for pertrochanteric fractures on the left side, inserted in a pertrochanteric fracture.

### Preferred Embodiment of the Invention

The attached drawing shows a preferred embodiment of the invention. More specifically, the surgical screw with an inverted thread for pertrochanteric fractures on the left side, object of the present specification, is characterized by comprising a body (1) having a series of notches made at one of its ends to allow performing a counterclockwise movement with an external tool, and characterized in that said body has a thread (2) at the other end in the inverted direction, such that the body must be introduced counterclockwise.

In a preferred embodiment, the body (1) will be introduced in a plate (3) that will be fixed to the bone (4) as a result of the presence of additional screws (5), as shown in Figure 1.

## Claims

1. A surgical screw with an inverted thread for pertrochanteric fractures on the left side comprising a body (1) having a series of notches made at one of its ends to allow performing a counterclockwise movement with an external tool for screwing it in, and **characterized in that** said body has a thread (2) at the other end in the inverted direction, such that the body must be introduced counterclockwise.
